(19) **Europäisches Patentamt European Patent Office Office européen des brevets**

(11) **EP 2 114 331 B1**

(12) **EUROPEAN PATENT SPECIFICATION**

(45) Date of publication and mention
of the grant of the patent:
**21.06.2017 Bulletin 2017/25**

(51) Int Cl.:
*A61Q 13/00* [(2006.01)]    *A61K 8/73* [(2006.01)]
*A61K 8/02* [(2006.01)]    *A61F 13/15* [(2006.01)]

(21) Application number: **08719529.3**

(22) Date of filing: **29.02.2008**

(86) International application number:
**PCT/IB2008/050753**

(87) International publication number:
**WO 2008/104960 (04.09.2008 Gazette 2008/36)**

(54) **PERSONAL CARE PRODUCT COMPRISING CYCLODEXTRIN AS FRGRANCE-COMPLEXING MATERIAL**

KÖRPERPFLEGEPRODUKT MIT CYCLODEXTRIN ALS DUFTKOMPLEXBILDENDEM MATERIAL

COMPOSITIONS COMPRENANT UN MATÉRIAU COMPLEXE À CYCLODEXTRINE

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **01.03.2007 US 712771**
**25.02.2008 US 36490**

(43) Date of publication of application:
**11.11.2009 Bulletin 2009/46**

(73) Proprietor: **The Procter & Gamble Company**
**Cincinnati, OH 45202 (US)**

(72) Inventors:
• **SCAVONE, Timothy, Alan**
**Loveland, Ohio 45140 (US)**
• **LEBLANC, Michael, Jude**
**Cincinnati, Ohio 45219 (US)**
• **SANKER, Lowell, Alan**
**Cincinnati, Ohio 45242 (US)**
• **SWITZER, Adrian, Gregory**
**Springboro, Ohio 45458 (US)**

(74) Representative: **Kremer, Véronique Marie Joséphine et al**
**Procter & Gamble Service GmbH**
**IP Department**
**Frankfurter Strasse 145**
**61476 Kronberg im Taunus (DE)**

(56) References cited:
**EP-A- 0 392 607    EP-A- 0 392 608**
**EP-B- 0 902 679    EP-B- 1 024 785**
**WO-A-00/76472**

Remarks:
The file contains technical information submitted after the application was filed and not included in this specification

**Description**

FIELD OF THE INVENTION

The present invention is directed to personal care products containing cyclodextrin complexing material and a fragrance material complexed with the same.

SUMMARY OF THE INVENTION

The present invention is directed to personal care products as defined in claim 1. The articles may include, but are not limited to, wipes, patches, and absorbent articles. Exemplary absorbent articles include diapers, feminine hygiene products, incontinence products, and wound dressings. The compositions and articles comprise a plurality of particles that include a cyclodextrin complexing material and a fragrance material complexed with the same. The particles may be manufactured to provide a high level of complexing efficiency. That is, a majority of the fragrance is bound to the interior of the cyclodextrin molecules, so that the perceptibility of the fragrance is minimized prior to its release. The particles may also have relatively low levels of moisture to help minimize the potential for microbial growth in the compositions or articles. Particles having relatively low levels of the moisture may also have a reduced tendency to agglomerate. Agglomerated particles can lead to a grainy or gritty feel associated with the composition or articles. Since the personal care products of the present invention include compositions that are applied to the body and articles that are worn/applied against the body, a grainy or gritty feel is likely undesirable for a significant number of users.

In accordance with some of the preferred embodiments, there has now been provided an article applied against the body comprising a cyclodextrin complexing material and a fragrance material, wherein the percent of the fragrance material that is complexed with the cyclodextrin is greater than about 90%, so that the perceptibility of the fragrance is minimized prior to its release, and wherein the composition does not contain an antiperspirant active.

In accordance with yet another preferred embodiment, there has now been provided a personal care product comprising a composition that is applied to the body or clothing, or an article applied against the body; and a plurality of particles associated with the composition or a component of the article, the plurality of particles comprising a cyclodextrin complexing material and a fragrance material, at least some of which being complexed with the cyclodextrin, wherein the plurality of particles has a moisture level, before their association with the composition or article component, of less than about 20% by weight of the particles, wherein the composition does not contain an antiperspirant active.

The above-described personal care products further comprise a second fragrance material that is not complexed with the cyclodextrin and that is different from the complexed fragrance.

The preferred embodiments described above explicitly exclude an antiperspirant active. However, other preferred embodiments can include such an active.

DETAILED DESCRIPTION OF THE INVENTION

The present invention may be understood more readily by reference to the following detailed description of illustrative and preferred embodiments. It is to be understood that the terminology used herein is for the purpose of describing particular embodiments by way of example only and it not intended to be limiting of the claimed invention. Also, as used in the specification including the appended claims, the singular forms "a," "an," and "the" include the plural, and reference to a particular numerical value includes at least that particular value, unless the context clearly dictates otherwise. When a range of values is expressed, another embodiment includes from the one particular value and/or to the other particular value. Similarly, when values are expressed as approximations, by use of the antecedent "about," it will be understood that the particular value forms another embodiment. All ranges are inclusive and combinable.

The personal care products of the present invention comprise cyclodextrin complexing material and a fragrance material complexed with the same.

When the personal care products are in the form of an article, the cyclodextrin-fragrance complexes may be loosely employed between two or more layers/components of the article and/or adhered to a layer or component of the article with a suitable adhesive, such as, for example, a styrene-based block copolymer.

The personal care products of the present invention are absorbent articles, for example, diapers, feminine hygiene products, incontinence products, and wound dressings. Absorbent articles typically include a liquid permeable top sheet or cover layer, a liquid impermeable back sheet or layer, and an absorbent core disposed therebetween. The articles may include additional components, such as, for example, a transfer layer underlying the top sheet that both facilitates quick fluid transfer from the top sheet to the absorbent core and deters fluid from leaving the absorbent core after the acquisition (i.e., deters "rewet" or "squeeze out"). Exemplary top sheets and transfer layers can include nonwovens, woven sheets, and apertured films. Exemplary absorbent cores can include wood pulp, hydrogels, absorbent polymers, and the like. And exemplary back sheets can include a polyolefin film. As noted above, the complexes may reside loosely between one or more of these absorbent article components and/or may be adhered to the same via an appropriate adhesive.

In some of the preferred embodiments of the present invention, the personal care products include a first fragrance material complexed with cyclodextrin, and a second uncomplexed fragrance material that is different from the first fragrance material. This design yields an initial scent expression with a different follow-up or secondary scent expression upon release of the second fragrance material.

Cyclodextrin Complexing Material

Personal care products of the present invention include a cyclodextrin complexing material for substantially "hiding" a fragrance material until a triggering mechanism has occurred, such as, for example, perspiration, urination, menstruation, to "release" the fragrance material. As used herein, the term "cyclodextrin" includes any of the known cyclodextrins such as unsubstituted cyclodextrins containing from about six to about twelve glucose units, especially alpha-cyclodextrin, beta-cyclodextrin, gamma-cyclodextrin and/or their derivatives and/or mixtures thereof. For example, the present invention may use cyclodextrins selected from the group consisting of beta-cyclodextrin, hydroxypropyl alpha-cyclodextrin, hydroxypropyl beta-cyclodextrin, methylated-alpha-cyclodextrin, methylated-beta-cyclodextrin, and mixtures thereof. Cyclodextrins may be included within the personal care products from at least about 0.1%, from at least about 1%, from at least about 2%, or from at least about 3% to about 25%, to about 20%, to about 15% or to about 10%, by weight of the composition or article component.

Cyclodextrin particles and cyclodextrin complexes comprising a fragrance material can be formed by various methods. For example, a solvent (e.g., water), unloaded cyclodextrin particles, and a fragrance material can be placed into a container and then mixed for a period of time to permit loading of fragrance molecules into "cavities" of cyclodextrin molecules. The mixture may or may not be processed further; e.g., processed through a colloid mill and/or homogenizer. The solvent is then substantially removed from the resulting mixture or slurry to yield cyclodextrin complex particles. Different manufacturing techniques may however impart different particle/complex characterizations, which may or may not be desirable in the personal care product. In accordance with some of the preferred embodiments of the present invention, the particles and/or complexes have a low level of moisture prior to their inclusion into the personal care product. For a given volume of cyclodextrin particles (at least some of which being complexed with a fragrance material), it is preferred to have a moisture level of less than about 20% by weight of the particles, more preferred to have a moisture level of less than about 10% by weight of the particles, and even more preferred to have a moisture level of less than about 6% by weight of the particles, prior to the inclusion of the volume of particles into the composition.

Spray drying a slurry or mixture of cyclodextrin-fragance complexes is the manufacturing technique capable of producing the cyclodextrin particles and cyclodextrin complexes having the above-noted, preferred moisture levels. Table I below provides a comparison of spray dried cyclodextrin complexes versus complexes formed via an extruder process (kneading).

Table I: Cyclodextrin Complex Moisture Level

| Sample | % Moisture |
| --- | --- |
| Spray Dry Process Sample A | 4.4 |
| Spray Dry Process Sample B | 3.7-4.5 |
| Spray Dry Process Sample C | 5.3 |
| Extruder Process Sample A | 27.87 |
| Extruder Process Sample B | 27.97 |
| Extruder Process Sample C | 24.00 |

Water content, USP (United States Pharmacopeia, current as of August 1, 2006) <921> Method I is the analytical method for determining cyclodextrin complex moisture level, as shown in Table I.

As one can see from Table 1, the moisture level directly manifested by these two methods is dramatically different. A kneading and extrusion method, or other method forming particles/complexes with higher than desired moisture levels, would require additional processing after their initial formation. For example, extruded complexes may require processing through an oven or dryer, or exposure to a controlled environment for a period of time.

Although not wishing to be bound by theory, it is believed that cyclodextrin particles/complexes having a relatively high moisture level have an increased tendency to agglomerate. The agglomerated particles may reach a size so as to become perceptible by a consumer; that is, a consumer may characterize the composition as being "gritty." And a "gritty" antiperspirant composition may not be desirable to some consumers, particular in solid product forms where the product is rubbed against the body as the means of applying the antiperspirant. Microbial growth is another potential disadvantage associated with employing cyclodextrin particles/complexes with relatively high moisture levels into a final composition depending on the remaining ingredients of the composition and/or storage parameters.

The efficiency or level of complexing with a fragrance material is another parameter of cyclodextrin complexes that can vary greatly depending on the manufacturing techniques employed. Put another way, the percent of fragrance material that is associated with the interior of a cyclodextrin molecule compared to the percent of fragrance material that is associated with the exterior of the cyclodextrin complex. The fragrance material that is on the exterior region of the complex is essentially free to be expressed without the requirement of a triggering mechanism, such as perspiration. The probability that a consumer perceives the fragrance material prior to a triggering mechanism increases as the level of free fragrance increases. And perception of a fragrance material prior to a triggering mechanism may not be desired depending on the overall composition design and targeted benefit associated with employment of the cyclodextrin complexes. In accordance with at least some of the preferred embodiments, the percent of fragrance material that is complexed with cyclodextrin is greater than about 75%, in some instances greater than about 90%, and in other instances greater than about 95%. It should be understood that these levels of fragrance complexation are directly associated with the complex formation process itself; the percentages do not represent a formulation design of adding a first percentage of fragrance material via a cyclodextrin complex and adding a second percentage of neat fragrance material.

Spray drying a slurry or mixture of cyclodextrin-fragance complexes is the manufacturing technique capable of producing cyclodextrin complexes having the above-noted levels of fragrance complexation. Table II below provides a comparison of spray dried cyclodextrin complexes versus complexes formed via an extruder process (kneading).

Table II: Percent of Fragrance Loading in Cyclodextrin Complexes

| Sample | Complexation Efficiency |
|---|---|
| Spray Dry Process Sample A | 96.6 |
| Spray Dry Process Sample B | 96.8 |
| Spray Dry Process Sample C | 96.2 |
| Extruder Process Sample A | 60.77 |
| Extruder Process Sample B | 65.47 |
| Extruder Process Sample C | 67.07 |

One can see from Table II that spray drying is capable of producing cyclodextrin complexes with very little free fragrance as compared to a kneading/extruder process. Additional processing steps may, for example, need to be employed to eliminate free fragrance associated with extruded complexes prior to their inclusion into a composition.

The analytical method for determining the percent of fragrance complexed, as shown in Table II, determines the free fragrance level in the complex by dissolving a sample in tetrahydrofuran (THF) adding an internal standard, and analyzing by capillary gas chromatography (GC). The complexed fragrance level is measured by extracting the same sample in acetone containing an internal standard, and analyzing by GC.

$$\text{Complexation Efficiency} = \% \text{ Complexed} / [\% \text{ Complexed} + \% \text{ Free}]$$

ORIGINAL SAMPLE PREPARATION

Internal Standard Stock Solution (ISSS)

Weigh 0.625 g $\pm$ 0.05g of Diphenyloxide into a tared 100 mL volumetric flask and make to volume with acetone (Baker HPLC grade 9254-03). This is a suggested internal standard, other materials may be substituted as necessary to avoid chromatographic overlap depending on the specific fragrance to be analyzed.

Standards

Select a sufficient number (typically 10-20) of fragrance components to account for 80% or greater of the total area of the fragrance chromatogram. A synthetic blend of these components will be the primary standard used to quantitate fragrance levels. A sample of the fragrance is used as the secondary standard which enables correction for the fact that less than 100% of the components are calibrated.

Primary Standard Calibration Solutions

Primary stock: Weigh 0.1 g (to 0.001g) of the individual fragrance components to be quantitated into a tared 100 mL volumetric flask and record the weights. Make to volume with acetone. Pipette 3.0 mL of the primary stock into a 50 mL volumetric flask and add 0.50 mL of ISSS for complexed calibration standard and dilute to volume with acetone. Pipette 3.0 mL of the primary stock into a 50 mL volumetric flask and add 0.50 mL of ISSS for neat calibration standard and dilute to volume with THF (Baker 9450-03).

Secondary Fragrance Standard Calibration Solutions

Secondary stock: Weigh 0.5 g ($\pm$0.1 g with precision to 0.0001 g) of the fragrance into a tared 100 mL volumetric flask and record weight. Make to volume with extraction solution for total fragrance (acetone); mix well. Pipette 3.0 mL of the secondary stock into a 50 mL volumetric flask and add 0.50 mL of ISSS for complexed fragrance standard and dilute to volume with acetone. Pipette 3.0 mL of the secondary stock into a 50 mL volumetric flask and add 0.50 mL of ISSS for neat fragrance standard and dilute to volume with THF.

Preparation of Samples

The ASE Solvent Extractor used in these analyses was a Dionex 200. Insert fiber filter (Dionex #49458) into an 11 mL cell body (Dionex part number 47004) with end cap on one end. Push filter to meet end cap. Tare on balance. Carefully add 1.000 gram (+/- 0.250 grams) of sample to cell and record actual weight. Using a funnel, add sand (30-40 mesh, EM Science EM-SX0075-1 or alternate inert material) to fill the cell, place another fiber filter on top and close cell with second end cap. Use care in applying this filter so it is not above the end of the cell but rather push down slightly so the filter is inside the walls of the cell. This is to avoid filter particles from accumulating within the threads of the end caps which can cause leaking during extraction. Record cell serial number to correspond with sample identification. Load the cells and their corresponding collection vials (60ml Dionex 48784) onto the ASE. [Note: For each sample two collection vials will be needed, one for the THF extraction (neat fragrance) and one for the acetone extraction (complex fragrance). To extract multiple samples it is recommended that all the THF extractions be done prior to the acetone extractions due to the temperature difference between the two methods.]

**ASE Methods**

| THF - Neat Fragrance | Extraction | Acetone - Complexed |
|:---:|:---:|:---:|
| | | |
| 0 min | Preheat | 0 min |
| 5 min | Heat | 6 min |
| 4 min | Static | 15 min |
| 100% | Flush | 100% |
| 60sec | Purge | 60sec |
| 1 | Cycles | 3 |
| 500 psi | Pressure | 2000 psi |
| 40°C | Temperature | 110°C |
| 100% THF | Solvent | 100% Acetone |

Preparation of ASE

Assure sufficient nitrogen flow by verifying pressures for solvent bottles are at 10 psi, system air is at 50 psi and compression oven is at 130 psi. Verify there is an adequate amount of nitrogen to complete the run. Typically 1000 psi of nitrogen is used to extract 15 samples. Enter ASE methods, above, and save each method under a separate number. For example: The THF method can be saved as number 1 and the acetone method can be saved as number 2. Verify there is an adequate volume of both THF and acetone present to complete the run. Approximately 30 mL of each solvent is used per sample (note: usage can vary from system to system). With rinse collection vials present and an adequate volume of THF present, rinse the system with THF a few times to prime lines and remove any air. With cells and their corresponding labeled collection vials in place, the ASE methods are ready to begin.

Post ASE Sample Preparation for Complexed Fragrance (in acetone)

Remove ASE collection vials containing complexed fragrance extract. Screw off the cap on the collection vial. Add 0.50mL of ISSS directly to the collection vial, with a volumetric pipet. Add approximately 30mL of acetone. Replace the cap onto the collection vial tightly. Shake well for approximately 30 seconds.

Post ASE Sample Preparation for Neat Fragrance (in THF)

Remove ASE collection vials containing neat fragrance extract. Screw off the cap on the collection vial. Add 0.50mL of ISSS directly to the collection vial, with a volumetric pipet. Add approximately 30mL of tetrahydrofuran. Replace the cap onto the collection vial tightly. Shake well for approximately 30 seconds.

APPARATUS CRITERIA (SUGGESTED TYPE OR SOURCE)

Gas Chromatograph HP5890 or equivalent equipped with capillary inlet system. and flame ionization detector with peak integration capabilities Column DB-5 column, 30 m x 0.32 mm I.D. with 1.0 micron coating, J&W Scientific cat. no. 123-5033

Gas Chromatographic Conditions

Carrier Gas Helium UHP grade or regular grade helium purified through a dry tube and an oxygen scrubber. Flow-pressure regulated at 15 psi with 30 mL/min. split flow.
Oven Temperature. 50°C-250°C @ 6°C/min; 250°C-315°C @ 70°C/min.; Hold at 315oC for 5 minutes

Injector Temperature 250°C
Detector Temperature 325°C
Hydrogen and Air Flows Optimized for gas chromatograph used
Integration Threshold 2, peak width 0.04
Injection 1 microliter: splitless mode

CALCULATIONS

$$\% \text{ Analyte} = [(\text{AvRf}) (A) (B) \times 100]/[C \times D]$$

Where:

AvRf = Average response factor for standard sample
A = Weight of internal standard added to sample solution
B = Area of analyte peak in sample chromatogram
C = Area of internal std. peak in sample chromatogram
D = Sample weight in gram
100 = Factor for percent conversion

Corrected % Complexed or % Free in samples = [sum of the % of all individual fragrance components in sample x 100] / [sum of the % of all individual fragrance components in the sec. std.]

The cyclodextrin complexes may be coated to minimize premature release/activation. Generally, any material that is capable of resisting water penetration is suitable. The coating material may include, for example, hydrocarbons, waxes, petrolatum, silicones, silicone derivatives, partially or fully esterfied sucrose esters, and polyglycerol esters. Using petrolatum as an example, a coating process may include combining cyclodextrin complexes with petrolatum at a ratio of about 1:1, for example, and then mixing until the complexes are satisfactorily coated. Another technique for delaying release or activation of a complexed fragrance, as contemplated herein, is to combine the fragrance material with an occlusive ingredient, such as, for example, coconut oil or petrolatum, before complexing with cyclodextrin. And the fragrance material and the cyclodextrin-fragrance complex may both be coated in some instances.

A scent-releasing system may be employed in the personal care products, wherien the system comprises cyclodextrin complexing material, as described above, in combination with other complexing or encapsulating materials known to the skilled artisan. For example, a scent-releasing system may be employed comprising a combination of cyclodextrin complexing material and one or more additional encapsulating materials. Exemplary encapsulating materials include starches, oligosaccharides, polyethylenes, polayamides, polystyrenes, polyisoprenes, polycarbonates, polyesters, polyacrylates, vinyl polymers, silicas, and aluminosilicates. Commercially available encapsulating materials N-Lok™, manufactured by National Starch, Narlex™ (ST and ST2), and Capsul E™ are useful for the present invention. These materials comprise pregelatinized waxy maize starch and optionally, glucose. The starch is modified by adding monofunctional substituted groups such as octenyl succinic acid anhydride. Accordingly, compositions of the present invention may include a neat fragrance material, a cyclodextrin-fragrance complex, and fragrance material encapsulated with materials other than cyclodextrin, such as those described above. The fragrances of this three-component scent-releasing system may be the same or different. Combining different scent-releasing technologies permits customization of scent expression profiles.

It should be understood that personal care products of the present invention may optionally employ "unloaded" cyclodextrin particles to act as a scavenger for malodor. These optional cyclodextrin particles may or may not have similar properties (or be manufactured using the same techniques) as the complexes described above.

Fragrance Material

Personal care products of the present invention may employ at least one fragrance material that is complexed with the cyclodextrin complexing material discussed above. As used herein, the term "fragrance material" includes both fragrant materials and odor controlling materials. That is, "fragrance material" includes not only perfumes, that is substances having a pleasant odor that mask or cover malodors, but also substances that may or many not have a pleasant odor per se but are able to improve the odor perception of the composition or article of which they are associated. Such odor controlling materials may act, for example, by modifying how certain nose receptors perceive malodors, or by acting on the malodorous substance by chemical reaction, or complexation, or absorption/adsorption, for example. Thus, in accordance with absorbent article embodiments of the present invention, the fragrant materials or odor controlling materials may improve the perceived odor of the absorbent article before, during, and/or after its use.

A representative, non-limiting, list of fragrance materials that may be complexed with the cyclodextrin includes anethole, benzaldehyde, decyl aldehyde, benzyl acetate, benzyl alcohol, benzyl formate, benzyl propionate, iso-bornyl acetate, camphene, cis-citral (neral), citronellal, citronellol, citronellyl acetate, paracymene, decanal, dihydrolinalool, dihydromyrcenol, methyl benzyl carbinyl acetate, dimethyl benzyl carbinyl acetate, dimethyl phenyl carbinol, eucalyptol, helional, geranial, geraniol, geranyl acetate, geranyl nitrile, cis-3-hexenyl acetate, dihydrocitronellal, d-limonene, linalool, linalool oxide, tetra-hydro linalool, alpha-methyl ionone, methyl nonyl acetaldehyde, methyl phenyl carbinyl acetate, laevo-menthyl acetate, menthone, iso-menthone, myrcene, myrcenyl acetate, myrcenol, nerol, neryl acetate, nonyl acetate, phenyl ethyl alcohol, phenyl acetaldehyde, alpha-pinene, beta-pinene, gamma-terpinene, terpineol, alpha-terpineol, beta-terpineol, terpinyl acetate, vertenex (para-tertiary-butyl cyclohexyl acetate), gamma-methyl ionone, undecalactone, undecylenic aldehyde, alpha-damascone, beta-damascone, amyl acetate, lemon oil, orange oil, and mixtures thereof.

Additional fragrant or odor controlling materials suitable for use in embodiments of the present invention include, but are not limited to, hexyl cinnamic aldehyde, alpha-amylcinnamic aldehyde, p-anisaldehyde, cinnamic aldehyde, cuminic aldehyde, p-t-butyl-alpha-methyldihydrocinnamaldehyde, 4-hydroxy-3-methoxycinnamaldehyde, 2-phenyl-3-(2-furyl)prop-2-enal, vanillin isobutyrate, ethyl vanillin acetate, vanillin acetate, cyclamen aldehyde, heptanal, lauryl aldehyde, nonanal, octanal, phenyl propyl aldehyde, vanillin, salycil aldehyde, cytral, 2,4-dihydroxy-3-methylbenzaldehyde, 2-hydroxy-4-methylbenzaldehyde, 5-methyl salicylic aldehydes, 4-nitrobenzaldehyde, o-nitrobenzaldehyde, 5-ethyl-2-thiophenecarbaldehyde, 5-methyl-2-thiophenecarboxaldehyde, 2-thiophenecarbaldehyde, asaronaldehyde, 5-(hydroxymethyl)-2-furaldehyde, 2-benzofurancarboxaldehyde, 2-benzofurancarboxaldehyde, 4-ethoxy-3-methoxy benzaldehyde, Protocatechualdehyde, Heliotropine, 2,3,4-trimethoxybenzaldehyde, 3,4,5-trimethoxybenzaldehyde, 2,8-dithianon-4-3n-4-carboxaldehyde, Sorbinaldehyde, 2,4-heptadienal, 2,4-decadienal, 2,4-nonadienal, (E,E)-,2,4-octadien-1-al, 2,4-octadienal, 2,4-dodecadienal, 4-undecadienal, 2,4-tridecadien-1-al, 2-trans-4-cis-7-cis-tridecatrienal, piperonylidene propionaldehyde, 2-methyl-3-(2-furyl)acrolein, 2,4-pentadienal, 2-furfurylidene butyraldehyde, 3-(2-furyl)acrolein, Pyruvaldehyde, Ethanedial, menthol, 3-buten-2-one, 3-methyl-4-(2,6,6-trimethyl-2-cyclohexen-1-yl), 4-(2,6,6-trimethylcyclohen-1-en-1-yl)but-3-en-2-one, 3-buten-2-one,4-(2,6,6-trimethyl-2-cyclohexen-1-yl)-, (E)-, menthyl lactate, isomenthyl acetate, isomenthyl propionate, isomenthyl isobutyrate, camphor, p-menthane, cresol, tetra hydromyrcenol, cytronellol, cytronellyil derivatives, geranyl derivatives, linalyl acetate, mugetanol, eugenol, jasmal, pinanol, cedrene, beta pinene, cineole, nonadienol, ethylhexanal, octanol acetate, methyl furfural, terpinene, thujene, amylacetate, benzylacetate, di-hydrocumarin, di-hydromyrcenyl acetate, Isoamylacetate, para-cymene, triethyl acetate, para-cresol, ethyl acetate, benzyl-benzoate, isopropyl myristate, methyl abietate, Ethanol, Isopropanol, diethyl sebacate, Glycerol, propylene glycol, 1,2-butylene glycol, dipropylene glycol, 2-methyl-2,4-pentanediol, diethylene glycol monoethyl ether, diethyl phthalate, hexyl salycilate, triethyl citrate, benzyl salicylate, and mixtures thereof. It may be desirable to include two or more fragrance materials in the personal care product, with at least one the fragrance materials being complexed with the cyclodextrin complexing material and at least one other fragrance material being added as a neat fragrance into the personal care product. In these embodiments, it is preferred for the complexed and neat fragrances to be different from one another. The differences can include types (including, for example, chemical make-up) and numbers of perfumes or other aromatic materials employed in the individual fragrance materials, the concentration level, or both.

The neat or non-complexed fragrance material may include the materials delineated above, or may include other perfumes/aromatic materials known to a person of ordinary skill in the art of creating fragrances. Typical fragrances are described in Arctander, Perfume and Flavour Chemicals (Aroma Chemicals), Vol. I and II (1969) and Arctander, Perfume and Flavour Materials of Natural Origin (1960). U.S. Patent No. 4, 322,308, issued to Hooper et al., March 30, 1982 and U.S. Patent No. 4,304,679, issued to Hooper et al., December 8, 1981 disclose suitable fragrance materials including, but not limited to, volatile phenolic substances (such as iso-amyl salicylate, benzyl salicylate, and thyme oil red), essence oils (such as geranium oil, patchouli oil, and petitgrain oil), citrus oils, extracts and resins (such as benzoin siam resinoid and opoponax resinoid), "synthetic" oils (such as BergamotTM 37 and BergamotTM 430, Geranium TM 76 and Pomeransol TM 314); aldehydes and ketones (such as B-methyl naphthyl ketone, p-t-butyl-A-methyl hydrocinnamic aldehyde and p-t-amyl cyclohexanone), polycyclic compounds (such as coumarin and beta-naphthyl methyl ether), esters (such as diethyl phthalate, phenylethyl phenylacetate, non-anolide 1:4).

## Claims

1. A personal care product, comprising:

   (a) an article applied against the body wherein the article is an absorbent article;
   (b) a plurality of particles associated with a component of the article, the plurality of particles being formed using a process comprising a step of spray drying and comprising a cyclodextrin complexing material and a first fragrance material, at least some of which being complexed with the cyclodextrin, wherein the plurality of particles has a moisture level, before their association with the article component, of less than about 20% by weight of the particles measured according to USP <921> Method I; and
   (c) a second fragrance material that is not complexed with the cyclodextrin and that is different from the first fragrance material.

2. The personal care product of claim 1, wherein the plurality of particles has a moisture level, before their association with the article component, of less than about 10% by weight of the plurality of particles.

3. The personal care product of claim 1, wherein the plurality of particles has a moisture level, before their association with the article component, of less than about 6% by weight of the plurality of particles.

4. The personal care product of claim 1, wherein the absorbent article is selected from the group consisting of diapers, feminine hygiene products, incontinence products, and wound dressings.

5. The personal care product of any preceding claim, wherein the percent of the first fragrance material that is complexed with the cyclodextrin is greater than about 90%, so that the perceptibility of the first fragrance is minimized prior to its release.

6. The personal care product of any preceding claim, wherein the percent of the fragrance material that is complexed with the cyclodextrin is greater than about 95%.

**Patentansprüche**

1.  Körperpflegeprodukt, umfassend:

    (a) einen Artikel, der an den Körper angelegt ist, wobei der Artikel ein Absorp-tionsartikel ist;
    (b) eine Vielzahl von Teilchen, die mit einem Bestandteil des Artikels verbunden ist, wobei die Vielzahl von Teilchen mittels eines Verfahrens gebildet wird, das einen Schritt des Sprühtrocknens umfasst, und ein Cyclo-dextrin-Komplexbildungsmaterial und ein erstes Duftstoffmaterial umfasst, von dem wenigstens ein Teil mit dem Cyclo-dextrin komplexiert ist, wobei die Vielzahl von Teilchen vor ihrer Verbindung mit dem Artikelbestandteil einen Feuchtigkeitsgehalt von weniger als etwa 20 Gew.-% der Teilchen, gemessen nach USP <921>, Verfahren I, aufweist; und
    (c) ein zweites Duftstoffmaterial, das nicht mit dem Cyclodextrin komplexiert ist, und das sich von dem ersten Duftstoffmaterial unterscheidet.

2.  Körperpflegeprodukt nach Anspruch 1, wobei die Vielzahl von Teilchen vor ihrer Verbindung mit dem Artikelbestandteil einen Feuchtigkeitsgehalt von weniger als etwa 10 Gew.-% der Vielzahl von Teilchen aufweist.

3.  Körperpflegeprodukt nach Anspruch 1, wobei die Vielzahl von Teilchen vor ihrer Verbindung mit dem Artikelbestandteil einen Feuchtigkeitsgehalt von weniger als etwa 6 Gew.-% der Vielzahl von Teilchen aufweist.

4.  Körperpflegeprodukt nach Anspruch 1, wobei der Absorptionsartikel ausgewählt ist aus der Gruppe bestehend aus Windeln, Damenhygieneprodukten, Inkontinenzprodukten und Wundverbänden.

5.  Körperpflegeprodukt nach einem der vorstehenden Ansprüche, wobei der Prozentsatz des ersten Duftstoffmaterials, das mit dem Cyclodextrin komplexiert ist, größer als etwa 90 % ist, so dass die Wahrnehmbarkeit des ersten Duftstoffs vor seiner Freisetzung minimiert ist.

6.  Körperpflegeprodukt nach einem der vorstehenden Ansprüche, wobei der Prozentsatz des Duftstoffmaterials, das mit dem Cyclodextrin komplexiert ist, größer als etwa 95 % ist.

**Revendications**

1.  Produit de soins d'hygiène personnelle, comprenant :

    (a) un article appliqué contre le corps ; dans lequel l'article est un article absorbant ;
    (b) une pluralité de particules associées à un composant de l'article, la pluralité de particules étant formée en utilisant un procédé comprenant une étape de séchage par atomisation et comprenant un matériau de complexation de type cyclodextrine et un premier matériau de parfum, dont au moins une partie est complexée avec la cyclodextrine, dans lequel la pluralité de particules a un taux d'humidité, avant leur association avec le composant d'article, inférieur à environ 20 % en poids des particules, mesuré selon le procédé I d'USP <921> ; et
    (c) un deuxième matériau de parfum qui n'est pas complexé avec la cyclodextrine et qui est différent du premier matériau de parfum.

2.  Produit de soins d'hygiène personnelle selon la revendication 1, dans lequel la pluralité de particules a un taux d'humidité, avant leur association avec le composant d'article, inférieur à environ 10 % en poids de la pluralité de particules.

3.  Produit de soins d'hygiène personnelle selon la revendication 1, dans lequel la pluralité de particules a un taux d'humidité, avant leur association avec le composant d'article, inférieur à environ 6 % en poids de la pluralité de particules.

4.  Produit de soins d'hygiène personnelle selon la revendication 1, dans lequel l'article absorbant est choisi dans le groupe constitué de couches, produits d'hygiène féminine, produits pour l'incontinence et pansements pour blessures.

5.  Produit de soins d'hygiène personnelle selon l'une quelconque des revendications précédentes, dans lequel le pourcentage du premier matériau de parfum qui est complexé avec la cyclodextrine est supérieur à environ 90 %, de sorte que la possibilité de perception du premier parfum est minimisée avant sa libération.

6. Produit de soins d'hygiène personnelle selon l'une quelconque des revendications précédentes, dans lequel le pourcentage du matériau de parfum qui est complexé avec la cyclodextrine est supérieur à environ 95 %.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4322308 A, Hooper

- US 4304679 A, Hooper

**Non-patent literature cited in the description**

- *Arctander, Perfume and Flavour Chemicals (Aroma Chemicals),* 1969, vol. I and II

- *Arctander, Perfume and Flavour Materials of Natural Origin,* 1960